# EUROPEAN PATENT APPLICATION

(11) **EP 4 699 596 A1**
(43) Date of publication of application: **25.02.2026**
(21) Application number: 24728686.7
(22) Date of filing: 22.04.2024
(51) Int. Cl.: A61J 17/00

(54) **PACIFIER**

(30) Priority: 21.04.2023 ES 202330688 U
(71) Applicant: Kahn, Sandra Vivian, San Mateo, CA 94402 (US)
(72) Inventor: Kahn, Sandra Vivian, San Mateo, CA 94402 (US)
(74) Representative: Isern Patentes y Marcas S.L.
(86) International application number: PCT/IB2024/053902
(87) International publication number: WO 2024/218757

(57) **Abstract**

The present invention provides a pacifier through which it can be detected if a user produces the negative pressure in the oral cavity necessary to hold the tongue in contact with the maxillary bone, stimulating the expansion thereof, so as to guide the correct growth of the face and the associated structures in the oral-nasal-respiratory complex. In addition, the pacifier serves as a guide for the eruption of deciduous teeth that begin to emerge from the gum, therefore being indicated to accompany the user in the different stages of growth.

## Description

### TECHNICAL FIELD OF THE INVENTION

The present invention relates to a pacifier of the type used by babies, where this pacifier has means that make it possible to visually demonstrate the presence of a vacuum pressure in the baby's oral cavity, in particular in the space of Donders, and also maintain said vacuum pressure to indicate and promote the correct functionality of the baby's nose and mouth.

### STATE OF THE ART

The use of a pacifier as a means of satisfying the sucking urge of babies has been used for many centuries. Therefore, in general, a pacifier is part of the usual items that a baby uses.

Thus, different complementary uses have been included in combination with the pacifier through the inclusion therein of means that allow stimulation actions for the baby to be carried out, for instance, reproducing musical sounds or the parents' voice, or supplying a substance or drug, among others.

On the other hand, pacifiers are also related to the development of the baby's sucking ability, which has a special impact on the development of the entire musculoskeletal structure of the face and the associated structures in the oral-nasal-respiratory complex.

Based on this fact, the review of scientific literature shows that the relationship between oral development and the efficiency of nasal breathing is extensive. Some studies have even shown that nasal breathing made difficult by a collapsed palate could be a cause of sudden infant death.

In accordance with the above, and given that the pacifier is an element commonly used by a baby that is part of its development, the need to implement means in a pacifier that allow parents and/or healthcare personnel to corroborate the correct functionality of the baby's nose and mouth, whether awake or, much more importantly, while sleeping, has been raised.

Based on the fact that correct functionality of the baby's nose and mouth will be that which generates and maintains negative pressure in the oral cavity (in the space of Donders) necessary to hold the tongue in contact with the maxillary bone, stimulating expansion thereof to guide the correct growth of the face and the associated structures in the oral-nasal-respiratory complex, the need to provide a pacifier that promotes the correct functionality of the baby's nose and mouth becomes evident.

### DESCRIPTION

To respond to the need found, according to a first aspect, the present invention provides a pacifier as disclosed in claim 1, wherein by means of the pacifier it can be detected if the user produces the negative pressure in the oral cavity necessary to hold the tongue in contact with the maxillary bone, stimulating the expansion thereof, so as to guide the correct growth of the face and the associated structures in the oral-nasal-respiratory complex.

According to the above, the proposed pacifier comprises a base plate, a cup hermetically couplable to the base plate and a nipple couplable to the base plate and/or the cup.

As is well known, the base plate is arranged to serve as a stop against the lips and prevent the user from swallowing the nipple.

On the other hand, in addition to the known function of providing the user with an element to suck, the nipple has an orthodontic design that allows a portion of same to be positioned between the tongue and the palate, specifically in the space of Donders, to record the negative pressure in said space, where suitable pressure is essential to hold the ideal position of the tissues within the mouth, nose and pharynx. A record of suitable negative pressure in the space of Donders is thereby indicative of suitable suction by the user, which, in turn, is indicative of the correct functionality of the nose and mouth of the user.

According to the above, the nipple is configured to establish fluid communication between the space of Donders of the oral cavity and the cup, such that the vacuum pressure in said space of Donders is transmitted to the cup.

In this sense, the cup has indicative means for indicating the pressure transmitted towards same which, in the preferred embodiment, take the form of a retractable region arranged in said cup, wherein said retractable region, under the influence of the aforementioned negative pressure, retracts, so it can be shown visually that the negative pressure in the space of Donders is sufficient and the suction is suitable. By transmitting the vacuum pressure to the cup, the latter helps maintain same, so it also assists in holding the tongue in contact with the palate, that is, the maxillary bone.

According to the above, the retractable region of the cup can be a membrane that conveniently retracts to provide visual feedback to any person that corroborates the correct functionality of the nose and mouth of the user, in particular a baby, who uses the Invention pacifier. A retraction of the retractable region indicates suitable effortless nasal breathing on the part of the user.

Optionally, in other alternative embodiments, the indicative means for indicating the pressure transmitted to the cup may be LED-type lights, or the vacuum pressure may be captured and the detection thereof transmitted to an external device, such as a computer, smartphone or the like, for subsequent treatment thereof.

With respect to the latter, the pacifier comprises an electronic unit that is linked to the cup, wherein said electronic unit is configured to receive an indicator signal indicating the retraction of the retractable region of the cup and/or the presence of a vacuum in the space of Donders, wherein the indicative means or indicator means are linked to said electronic unit to indicate the retraction of the retractable region and/or the presence of a vacuum in the space of Donders captured by said electronic unit.

In an alternative embodiment, the electronic unit is arranged on the base plate and comprises a sensor configured to detect the retraction of the retractable region and/or the presence of a vacuum in the space of Donders. Preferably, the sensor is a switch that is activated when the retractable region retracts, such that the activation of said sensor activates the indicator means which, as mentioned, can be LED lights that are selectively turned on or off, or an auditory signal, etc. Even more preferably, the indicator means are LED lights that are arranged in the electronic unit.

In another embodiment, the pacifier comprises a casing intended for the arrangement of the electronic unit and a flexible type tube designed to fluidically connect the cup with the casing, wherein the electronic unit has, in addition to the sensor for detecting the retraction of the retractable region and/or the vacuum pressure, processing means to process detection data, storage means to store said data and communication means to transmit same for further processing or analysis, or to download it in editable formats. In preferred embodiments, this casing takes the form of a clip intended to be attached to a user's garment.

On the other hand, the nipple comprises a proximal end through which it is coupled to the base plate and a distal end intended to be arranged in the space of Donders of the oral cavity. Preferably the nipple is hollow and comprises at least one hole arranged at the distal end, such that said at least one hole transmits the vacuum pressure from the space of Donders to the cup causing the retraction of the retractable region. It is important to note that the design of the position of the hole (or holes) on the nipple is critical to measure only and solely within the space of Donders without any other influence, such as positive pressure of the tongue on the palate, etc.

At this point it is noted that the base plate comprises a housing configured for coupling the proximal end of the nipple, and a connection port intended for coupling the cup, wherein the housing and the connection port are arranged on the base plate whereby fluid communication between the nipple and the cup is enabled. Furthermore, both the housing and the connection port are configured for the sealed coupling of said nipple and said cup to the base plate, such that the vacuum pressure is completely transmitted to the cup to show its presence visually.

In addition to the above, the pacifier comprises an intraoral screen configured to be interposed between the outer portion of the teeth and the inner portion of the lips and cheeks. The incorporation of this dental screen aims to serve as a guide for the deciduous teeth that begin to emerge from the gum, without generating malocclusions, allowing the transition from the needs of a toothless mouth of a newborn who is on a liquid diet (breast milk/ formula) towards a solid diet, therefore supporting weaning.

This screen, like the rest of the pacifier components, is made from hypoallergenic components and can be adapted according to the oral morphology of the Frankel space of each user, where the Frankel space is defined as the space between the teeth and the lips and cheeks.

In a second embodiment, the present invention provides a pacifier comprising a base plate and a cup, with a retractable region, hermetically couplable to the base plate, such as in the previous embodiment. In contrast to the previous embodiment, the pacifier comprises an intraoral screen, which is couplable to the base plate and/or to the cup, wherein said intraoral screen is in fluid communication with the cup, the intraoral screen being configured to be interposed between the outer portion of the teeth and the inner portion of the lips and cheeks, serving as a guide for the eruption of deciduous teeth that begin to emerge from the gum, wherein a vacuum pressure from the oral cavity is transmitted to the cup, causing the region retractable to retract.

In this second embodiment, the user creates a suitable vacuum in the space of Donders thanks to, for instance, having used the pacifier of the first embodiment. In this way, suction is suitable and, following the normal stage of development and promoting the growth of tissues, bones and eruption of deciduous teeth, the nipple is removed. In this way, the retractable region of the cup, under the influence of the vacuum pressure of the oral cavity, will contract, visibly showing said suitable suction. By transmitting the vacuum pressure to the cup, the latter helps maintain same, so it also assists in holding the tongue in contact with the palate, that is, the maxillary bone.

The intraoral screen comprises a tube projecting therefrom, the tube having open ends to define a channel through which fluid communication with the cup is established. In this sense, when the intraoral screen is located in the oral cavity, occupying the Frankel space, one open end is oriented towards the inside of the oral cavity, while the other end is oriented towards the cup, wherein the latter is hermetically coupled at said end.

Alternatively, in this second embodiment, it is possible to provide a nipple couplable to the intraoral screen, the nipple being configured to establish fluid communication between the space of Donders of the oral cavity and the cup, such that a vacuum pressure in said space of Donders causes the retractable region of the cup to retract.

This embodiment is possible, since the user may require the nipple at some point during the weaning process.

The nipple therefore comprises a proximal end through which it is coupled to the base plate and a distal end intended to be arranged in the space of Donders of the oral cavity.

Preferably the nipple is hollow and comprises at least one hole arranged at the distal end, such that said at least one hole transmits the vacuum pressure from the space of Donders to the cup causing the retraction of the retractable region.

### BRIEF DESCRIPTION OF THE FIGURES

The foregoing and other advantages and features will be more fully understood from the following detailed description of exemplary embodiments with reference to the accompanying drawings, which should be considered by way of illustration and not limitation, wherein:
- Figure 1 is a perspective view of the first embodiment of the pacifier in which the nipple can be seen.
- Figure 2 is a perspective view of the first embodiment of the pacifier from another point of view.
- Figure 3 is a perspective view of the first embodiment of the pacifier in which the incorporation of the intraoral screen in combination with the nipple can be seen.
- Figure 4 is a perspective view of the first embodiment of the pacifier in which the incorporation of the intraoral screen in combination with the nipple can be seen from another point of view.
- Figure 5 is a perspective view of the second embodiment of the pacifier in which the intraoral screen can be seen.
- Figure 6 is a perspective view of the second embodiment of the pacifier in which the intraoral screen can be seen from another point of view.
- Figure 7 is a perspective view showing the components of the pacifier in an exploded state.
- Figure 8 is a perspective view showing the components of the pacifier in an exploded state, and an electronic unit is further shown.
- Figure 9 is a view of an alternative embodiment in which a casing is coupled by means of a flexible type tube to the cup.

### DETAILED DESCRIPTION OF AN EXEMPLARY EMBODIMENT

In the following detailed description, numerous specific details are set forth in the form of examples to provide a thorough understanding of the relevant teachings. However, it will be apparent to those skilled in the art that the present teachings can be implemented without such details.

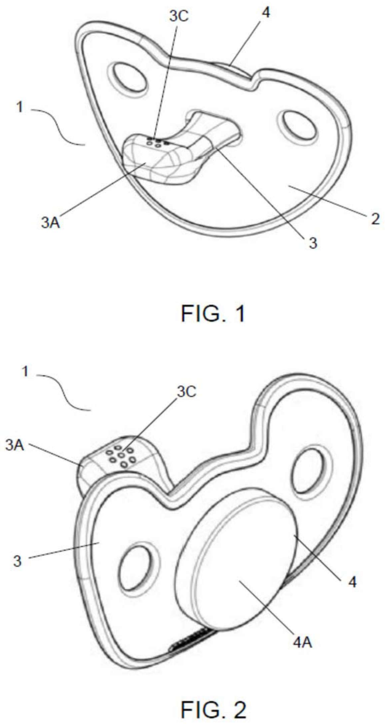

As seen in Figures 1 to 4, in a preferred embodiment of the first embodiment, the invention discloses a pacifier 1 intended to be used by a baby comprising a base plate 2, a cup 4 and a nipple 3.

Within the scope of this detailed description, the words "user", "baby" or "infant" will be used interchangeably to refer to the users for whom the pacifier 1 is intended, taking into account that its use is primarily intended for babies between 0 to 18 months of life.

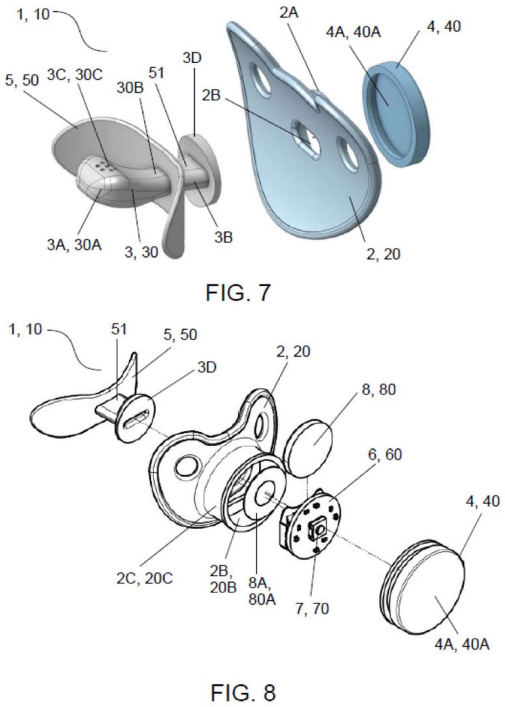

The base plate 2 comprises a connection port 2A intended for coupling the cup 4, where in this embodiment the coupling is carried out in a sealed manner by means of a snap-fit coupling, such that the cup 4 is releasable from the connection port 2A, as seen in Figure 7.

The base plate 2 also comprises a housing 2B, in the form of a through hole, intended for the sealed coupling of the nipple 3, wherein this housing 2B coincides with the connection port 2A to enable fluid communication between the nipple 3 and the cup 4.

Likewise, the nipple 3 is hollow 3 and has an orthodontic design that, in addition to providing the suction function, allows it to be positioned between the baby's tongue and palate and includes a proximal end 3B through which it is hermetically coupled to the base plate 2, in particular to the housing 2B, the proximal end 3B having a projection 3D which, once the proximal end 3B has been coupled to the housing 2B, prevents involuntary disconnection of the nipple 3. The nipple 3 also comprises a distal end 3A to be arranged between the baby's tongue and the palate, especially in the space of Donders of the oral cavity. A plurality of holes 3C are arranged at said distal end 3A, such that the holes 3C, given the hollow configuration of the nipple 3, transmit the vacuum pressure from the space of Donders to the cup 4, causing the retraction of a retractable region 4A of said cup 4.

As can be seen, in this preferred embodiment, the cup 4 has a tubular cylindrical shape with a closed end as a cover, wherein the retractable region 4A is arranged in said cover. The stiffness of the cover will be selected whereby the retractable region 4A thereof conveniently retracts under the influence of the vacuum pressure. Preferably, the retractable region 4A is the entire cover and consists of a flexible membrane, prepared to retract under the influence of the vacuum pressure and return to its original state when released from the vacuum pressure. Therefore, the materials from which the cup 4 is manufactured, in particular the retractable region 4A, will be selected whereby when the retractable region 4A retracts, this retraction is visually observable by a person, for instance, medical personnel or the baby's parents.

Therefore, when the baby uses the pacifier 1, the distal end 3A of the nipple 3 is placed in the space of Donders, capturing its vacuum and showing same by the retraction of the retractable region 4A of the cup 4.

As seen in Figure 8, in the preferred embodiment of this first embodiment, the pacifier 1 comprises a sleeve 2C projecting from the base plate 2, wherein the connection port 2A is arranged opposite the base plate 2 and wherein the housing 2B is arranged at least partially inside said sleeve 2C. An electronic unit 6 is configured to be arranged inside said sleeve 2C, wherein the electronic unit 6 is configured to be linked with the cup 4 such that it receives the retraction of the retractable region 4A of said cup 4. In the illustrated embodiment, the electronic unit 6 comprises a sensor 7, in the form of a switch, which is actuated by the retraction of the retractable region 4A under the presence of a vacuum pressure in the oral cavity. In addition to showing the presence of a vacuum through the retractable region 4A, the electronic unit 6 comprises LED lights (not illustrated) connected to the sensor 7, that is, to the switch, and an energy accumulator 8 in the form of a battery to power and energise the LEDs when they are activated by the switch. In this way, the user is shown the presence of a vacuum in the space of Donders by means of the retraction of the cup 4 and also by the illumination of the LEDs.

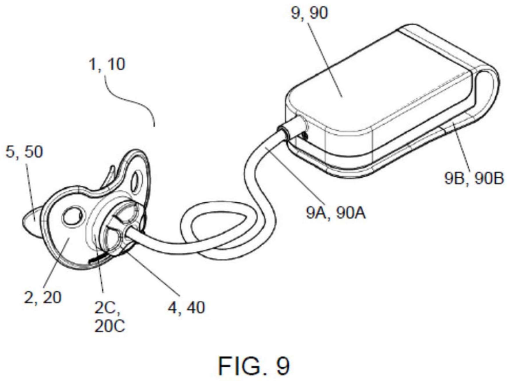

In another alternative embodiment, illustrated in Figure 9, the electronic unit 6 is arranged in a casing 9, wherein said casing 9 is fluidically connected to the cup 4 by means of a flexible type tube 9A. In addition to the sensor 7 for detecting the retraction of the retractable region and/or the vacuum pressure, the electronic unit 6 has processing means (not illustrated) for processing detection data from the sensor 7, storage means (not illustrated) for storing said data and communication means (not illustrated) to transmit same for further processing or analysis, or to download it in editable formats. As seen in Figure 9, preferably the casing 9 takes the form of a clip intended to be attached to a user's garment. In this sense, the clip has an opening 9B for coupling to the garment.

In addition to the above, a membrane 8A, preferably of the Goretex^{®} type, is arranged inside the sleeve 2C, configured to prevent fluids from the user's oral cavity from contacting the control unit 6.

Likewise, alternatively, the connection between the cup 4 and the base plate 2 through the connection port 2A may be of a threaded type.

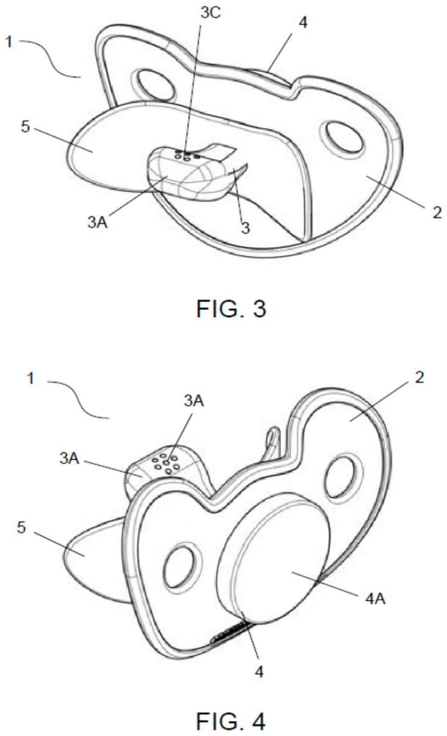

In a variation of this first embodiment, as seen in Figures 3 and 4, the pacifier 1 comprises an intraoral screen 5 couplable to the nipple 3, in particular in an area near the proximal end 3A, said intraoral screen 5 being intended for being interposed between the outer portion of the teeth and the inner portion of the lips and cheeks, in the so-called Frankel space, and configured to serve as a guide in the eruption of deciduous teeth that begin to emerge from the gum.

The intraoral screen 5 is made from a soft touch polymeric material, such as soft silicone, which can be cut and adapted to the size of the baby's mouth. As mentioned, the intraoral screen is intended to occupy the Frankel space, which is the labial vestibule. This space is the area between the outer portion of the teeth (or where they are expected to be when they come in) and the inner portion of the lips and cheeks. The implementation of this intraoral screen 5 in the pacifier 1 allows the teeth to erupt and develop suitably without generating malocclusions.

The intraoral screen 5 may optionally be couplable to the nipple 3, or can form a single piece with the latter.

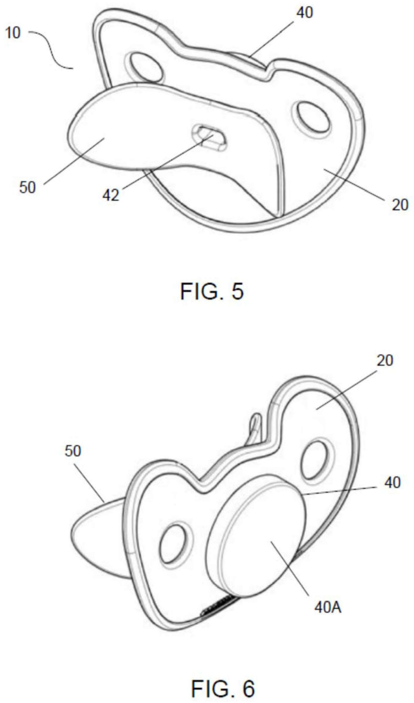

On the other hand, a second embodiment of the invention is illustrated in Figures 5 and 6, showing a pacifier 10 for a more advanced stage of development, in which weaning is intended and, therefore, the nipple us removed. In this way, the pacifier 10 in this second embodiment comprises a base plate 20, a cup 40 hermetically couplable to the base plate 20, the cup 40 comprising a retractable region 40A, and an intraoral screen 50 couplable to the base plate 20, wherein said intraoral screen 50 is in fluid communication with the cup 40 by means of a channel 52 that communicates these two elements, the intraoral screen 50 being configured to be interposed between the outer portion of the teeth and the interior portion of the lips and the cheeks, occupying the Frankel space, and to serve as a guide for the eruption of deciduous teeth that begin to emerge from the gum and prevent malocclusions, such that a vacuum pressure from the oral cavity is transmitted to the cup 40 causing the retractable region 40A to retract.

The vacuum shown by the retraction of the retractable region 40A of the cup 40 is generated by the suction in the oral cavity by the baby, wherein the maintenance of this vacuum is an indication of the proper working of the airways and the proper development of all the associated morphological structures, that is, the tissues within the mouth, nose and pharynx, and mandibular growth.

As seen in Figure 7, the intraoral screen 50 comprises a tube 51 projecting therefrom, the tube 51 having open ends to define a channel 52, observed in Figures 5 and 6, through which fluid communication with the cup 40 is established.

It is important to note that the base plate 20 and the cup 40 of this second embodiment have the same shape and function described for their respective analogues of the first embodiment. Therefore, the description made for such elements in the first embodiment of the invention is extended to these.

Optionally, in the pacifier 10, it is possible to incorporate a nipple 30 which will be couplable to the intraoral screen 50, the nipple 30 being configured to establish fluid communication between the space of Donders of the oral cavity and the cup 40, such that a vacuum pressure in said space of Donders will cause the retractable region 40A of the cup 40 to retract, making the vacuum visually evident.

The nipple 30 comprises a proximal end 30B through which it is coupled to the intraoral screen 50, in particular at the entrance of the channel 52, and a distal end 30A intended to be arranged in the space of Donders.

Furthermore, the nipple 30 is hollow and comprises a plurality of holes 30C arranged at the distal end 30A, such that the holes 30C transmit, through the nipple 30, the vacuum pressure from the space of Donders to the cup 40, causing the retraction of the retractable region 40A.

Likewise, within this second embodiment, analogously to the first embodiment, as observed in Figure 8, the pacifier 10 comprises a sleeve 20C projecting from the base plate 20, wherein the connection port 20A is arranged opposite the base plate 20 and wherein the housing 20B is arranged at least partially inside said sleeve 20C. An electronic unit 60 is configured to be arranged inside said sleeve 20C, wherein the electronic unit 60 is configured to be linked with the cup 40 such that it receives the retraction of the retractable region 40A of said cup 40. In the illustrated embodiment, the electronic unit 60 comprises a sensor 70, in the form of a switch, which is actuated by the retraction of the retractable region 40A under the presence of a vacuum pressure in the oral cavity. In addition to showing the presence of a vacuum through the retractable region 40A, the electronic unit 60 comprises LED lights (not illustrated) connected to the sensor 70, that is, to the switch, and an energy accumulator 80 in the form of a battery to power and energise the LEDs when they are activated by the sensor 70. In this way, the user is shown the presence of a vacuum in the space of Donders by means of the retraction of the cup 4 and also by the illumination of the LEDs.

In another alternative embodiment of this second embodiment, illustrated in Figure 9, the electronic unit 60 is arranged in a casing 90, wherein said casing 90 is fluidically connected to the cup 40 by means of a flexible type tube 90A. In addition to the sensor 70 for detecting the retraction of the retractable region 40A and/or the vacuum pressure, the electronic unit 60 has processing means (not illustrated) for processing detection data from the sensor 70, storage means (not illustrated) for storing said data and communication means (not illustrated) to transmit same for further processing or analysis, or to download it in editable formats. As seen in Figure 9, preferably the casing 90 takes the form of a clip intended to be attached to a user's garment. In this sense, the clip has an opening 90B for coupling to the garment.

In addition to the above, a membrane 80A, preferably of the Goretex^{®} type, is arranged inside the sleeve 20C, configured to prevent fluids from the user's oral cavity from contacting the control unit 60.

Likewise, alternatively, the connection between the cup 40 and the base plate 20 through the connection port 20A may be of a threaded type.

## Claims

1. A pacifier (1) comprising:
- a base plate (2);
- a cup (4) hermetically couplable to the base plate (2), the cup (4) comprising a retractable region (4A); and
- a nipple (3) couplable to the base plate (2) and/or to the cup (4), the nipple (3) being configured to establish fluid communication between the space of Donders of the oral cavity and the cup (4), such that a vacuum pressure in said space of Donders causes a retraction of the retractable region (4A) of the cup (4).

2. The pacifier (1) according to claim 1, wherein the nipple (3) comprises a proximal end (3B) through which it is coupled to the base plate (2) and a distal end (3A) intended to be arranged in the space of Donders of the oral cavity.

3. The pacifier (1) according to claim 2, wherein the nipple (3) is hollow and comprises at least one hole (3C) arranged in the distal end (3A), such that said at least one hole (3C) transmits, through of the nipple (3), the vacuum pressure from the space of Donders to the cup (4) causing the retraction of the retractable region (4A).

4. The pacifier (1) according to any of the preceding claims comprising an intraoral screen (5) couplable to the nipple (3) intended to be interposed between the outer portion of the teeth and the inner portion of the lips and cheeks, and configured to serve as a guide for the eruption of deciduous teeth that begin to emerge from the gum.

5. The pacifier (1) according to any of the preceding claims comprising an electronic unit (6) linked to the cup (4), said electronic unit (6) being configured to receive an indicator signal indicating the retraction of the retractable region (4A) and/or the presence of a vacuum in the space of Donders, and indicator means linked to said electronic unit (6) configured to indicate the retraction of the retractable region (4A) and/or the presence of a vacuum in the space of Donders.

6. The pacifier (1) according to claim 5, wherein the electronic unit (6) is arranged on the base plate (2) and comprises a sensor (7) configured to detect the retraction of the retractable region (4A) and/or the presence of a vacuum in the space of Donders.

7. The pacifier (1) according to claim 5 comprising a casing (9) intended for the arrangement of the electronic unit (6) and a tube (9A) of the flexible type configured to fluidically connect the cup (4) with the casing (9), wherein the electronic unit (6) comprises a sensor for detecting the retraction of the retractable region (4A) and/or the vacuum pressure, processing means, storage means and communication means.

8. A pacifier (10) comprising:
- a base plate (20);
- a cup (40) hermetically couplable to the base plate (20), the cup (40) comprising a retractable region (40A); and
- an intraoral screen (50) couplable to the base plate (20) and/or to the cup (40), wherein said intraoral screen (50) is in fluid communication with the cup (40), the intraoral screen (50) being configured to be interposed between the outer portion of the teeth and the inner portion of the lips and cheeks and to serve as a guide for the eruption of deciduous teeth that begin to emerge from the gum, such that a vacuum pressure from the oral cavity is transmitted to the cup (40), causing the retractable region (40A) to retract.

9. The pacifier (10) according to claim 8, wherein the intraoral screen (50) comprises a tube (51) projecting therefrom, the tube (51) having open ends to define a channel (52) through which fluid communication with the cup (40) is established.

10. The pacifier (10) according to any of claims 8 or 9, comprising a nipple (30) couplable to the intraoral screen (50), the nipple (30) being configured to establish fluid communication between the space of Donders of the oral cavity and the cup (40), such that a vacuum pressure in said space of Donders causes the retractable region (40A) of the cup (40) to retract.

11. The pacifier (10) according to claim 10, wherein the nipple (30) comprises a proximal end (30B) through which it is coupled to the base plate (20) and a distal end (30A) intended to be arranged in the space of Donders of the oral cavity.

12. The pacifier (10) according to claim 11, wherein the nipple (30) is hollow and comprises at least one hole (30C) arranged in the distal end (30A), such that said at least one hole (30C) transmits, through of the nipple (30), the vacuum pressure from the space of Donders to the cup (40) causing the retraction of the retractable region (40A).

13. The pacifier (10) according to any of claims 8 to 12 comprising an electronic unit (60) linked to the cup (40), said electronic unit (60) being configured to receive an indicator signal indicating the retraction of the retractable region (40A) and/or the presence of a vacuum in the space of Donders, and indicator means linked to said electronic unit (60) configured to indicate the retraction of the retractable region (40A) and/or the presence of a vacuum in the space of Donders.

14. The pacifier (10) according to claim 13, wherein the electronic unit (60) is arranged on the base plate (20) and comprises a sensor (70) configured to detect the retraction of the retractable region (40A) and/or the presence of a vacuum in the space of Donders.

15. The pacifier (1) according to claim 13 comprising a casing (90) intended for the arrangement of the electronic unit (60) and a tube (90A) of the flexible type configured to fluidically connect the cup (40) with the casing (90), wherein the electronic unit (60) comprises a sensor for detecting the retraction of the retractable region (40A) and/or the vacuum pressure, processing means, storage means and communication means.
